# EUROPÄISCHE PATENTSCHRIFT

(11) **EP 2 464 288 B1**
(45) Veröffentlichungstag und Bekanntmachung des Hinweises auf die Patenterteilung: **05.06.2013**
(21) Anmeldenummer: 10747162.5
(22) Anmeldetag: 05.08.2010
(51) Int. Cl.: A61B 5/083, A61B 5/22

(54) **VERFAHREN ZUR STEUERUNG EINES SPIROERGOMETRIESYSTEMS UND SPIROERGOMETRIESYSTEM**
METHOD FOR CONTROLLING A SPIROERGOMETRIC SYSTEM AND SPIROERGOMETRIC SYSTEM
PROCÉDÉ DE COMMANDE D'UN SYSTÈME DE SPIROERGOMÉTRIE ET SYSTÈME DE SPIROERGOMÉTRIE

(30) Priorität: 13.08.2009 DE 102009037038; 16.09.2009 DE 102009041425
(43) Veröffentlichungstag der Anmeldung: 20.06.2012
(73) Patentinhaber: Aceos GmbH, 90763 Fürth (DE)
(72) Erfinder: JUNG, Gunnar, 90763 Fürth (DE); TUCHTENHAGEN, Dirk, 90762 Fürth (DE); SCHOTTERS, Markus, 01099 Dresden (DE); KUSCH, Martin, 26180 Rastede (DE)
(74) Vertreter: Von Rohr
(86) Internationale Anmeldenummer: PCT/EP2010/004805
(87) Internationale Veröffentlichungsnummer: WO 2011/018187

(56) Entgegenhaltungen:
- EP-A1- 1 825 888
- GB-A- 2 162 642

## Beschreibung

Die Erfindung betrifft ein Verfahren zur Steuerung eines Spiroergometriesystems zur Bestimmung des Energiestoffwechsels während körperlicher Belastung und ein entsprechend ausgebildetes Spiroergometriesystem.

Bei der klassischen Spiroergometrie werden Atemgase eines Probanden während körperlicher Belastung gemessen. Die wichtigsten Atemgasparameter, die bei der Spiroergometrie erfaßt werden, sind die Sauerstoffaufnahme, die Kohlenstoffdioxidabgabe, das Atemminutenvolumen und die Atemfrequenz. Daraus lassen sich weitere Parameter berechnen, wie beispielsweise der respiratorische Quotient, das Atemäquivalent für Sauerstoff und/oder für CO₂ und das Atemzugvolumen.

Mit Hilfe von Parametern wie z. B. der maximalen Sauerstoffaufnahme während ansteigender körperlicher Belastung, der aeroben- und der anaeroben Schwelle lassen sich Aussagen über die Ausdauerleistungsfähigkeit eines Probanden treffen.

Aufgrund der stöchiometrischen Beziehungen zwischen Gasaustausch (Sauerstoffverbrauch, Kohlenstoffdioxidproduktion) und Substratstoffwechsel können die jeweiligen Substratoxidationsraten der Kohlenhydratoxidation und der Lipidoxidation auf der Grundlage der bestimmten Menge des bei einem Atemzug aufgenommenen Sauerstoffs und der Menge des anschließend ausgeatmeten Kohlenstoffdioxids ermittelt werden. Diese Zusammenhänge sind dem Fachmann als indirekte Kalometrie bekannt. Die Spiroergometrie kann somit zur Messung des Energiestoffwechsels während körperlicher Belastung zur Ermittlung von Kalorienverbrauch und Substratumsatz (Fett- und Kohlenhydratstoffwechsel) des Probanden eingesetzt werden. Darüber hinaus lassen sich durch einen Vergleich mit Normwerten aus den Ergebnissen der Spiroergometrie Rückschlüsse auf die Leistungsfähigkeit des cardiopulmonalen Systems des Probanden ziehen.

Die maximale Sauerstoffaufnahme gilt als klassischer Parameter zur Beurteilung der Ausdauerleistungsfähigkeit. Zusätzlich wird der Parameter der maximalen Sauerstoffaufnahme genutzt, um individuelle Trainingsintensitäten festzulegen. Hierbei werden Standardprozentbereiche als eine Art Filter über individuell gemessene Werte der maximalen Sauerstoffaufnahme gelegt, um einen "individuellen" Grundlagentrainingsbereich festzulegen. Dies führt zu einer Verallgemeinerung und zu ungenauen Trainingsempfehlungen. Unter Berücksichtigung von statistischen Werten wird beispielsweise der Grundlagentrainingsbereich des Menschen stets bei ca. 65 % der maximalen Sauerstoffaufnahme erreicht, was keinesfalls den tatsächlich individuellen Grundlagentrainingsbereich beschreibt und keine zielgerichtete Trainingsempfehlung zuläßt. Bei der Bestimmung der maximalen Sauerstoffaufnahme wird der Proband maximal ausbelastet. Die maximale Sauerstoffaufnahme ist dabei abhängig von der Ventilation, der Herz-Kreislauf-Kapazität und der lokalen Sauerstoffausschöpfung in der Muskulatur. Der Proband muß somit in der Lage sein, sich bis zur vollständigen Erschöpfung zu belasten. Bestimmte Krankheitsbilder schließen eine maximale Belastung jedoch von vornherein aus.

Bei aus dem Stand der Technik bekannten Verfahren zur Atemgasmessung mit einem Spiroergometriegerät ist es erforderlich, dass der Proband während der Messung eine Gesichtsmaske trägt, an die ein Volumenstromsensor zur Messung des ventilierten Luftvolumens sowie ein dünner Schlauch als Absaugstrecke angeschlossen ist. Über den Schlauch wird eine Gasprobe aus der Expirationsluft entnommen und zu Gassensoren geleitet, um die Konzentrationen der Gaskomponenten zu ermitteln. Der prozentuale Gasgehalt der Expirationsluft wird dann mit dem der Umgebungsluft verglichen. Die Berechung absoluter Werte ist bei Kenntnis des ventilierten Luftvolumens möglich. Auf der Grundlage der gemessenen Werte erfolgt dann die Bestimmung von physiologischen Parametern des Energiestoffwechsels des Probanden. Diese Abläufe und entsprechende Sensoren zur Atemgasanalyse sind dem Fachmann grundsätzlich bereits bekannt.

Da zur Ermittlung der maximalen Sauerstoffaufnahme die Atemgaszusammensetzung und das Atemflussvolumen kontinuierlich gemessen werden, ist es erforderlich, dass der Proband über die gesamte Dauer des Tests von ca. 8 bis 30 Minuten die Gesichtsmaske trägt, was in der Regel als störend empfunden wird. Im übrigen ist die lange Testdauer bis zum Erreichen einer maximalen Ausbelastung zur Bestimmung der maximalen Sauerstoffaufnahme von Nachteil und sehr anstrengend für den Probanden.

Aus der WO 2009/056457 A1 ist ein Verfahren zur Steuerung und/oder Regelung einer Trainings- oder Rehabilitationseinheit bekannt, wobei mit Hilfe einer Sensor-Einheit Atemgaszusammensetzungen und/oder Atemflussvolumina gemessen und auf deren Grundlage physiologische Parameter der Ventilation und/oder des Gasaustausches eines Probanden bestimmt werden können. Bei dem bekannten Verfahren ist vorgesehen, Leistungskenngrößen auf der Grundlage der bestimmten physiologischen Parameter bei submaximaler Belastung mit Hilfe einer Regressionsfunktion und/oder durch Ausbelastung bis zur maximalen Leistungsfähigkeit kontinuierlich zu ermitteln. Ein anderes Verfahren zur Steuerung und/oder Regelung einer Trainings- und/oder Rehabilitationseinheit ist aus der WO 2009/024427 A1 bekannt.

Weiter sind aus der EP 1 825 888 Intermittierende Messungen bekannt.

Aufgabe der vorliegenden Erfindung ist es, ein Verfahren zur Steuerung eines Spiroergometriesystems und ein Spiroergometriesystem jeweils der eingangs genannten Art zur Verfügung zu stellen, mit denen eine individuelle Leistungs- und Stoffwechseldiagnostik, insbesondere die Erstellung zielgerichteter individueller Ernährungs-, Bewegungs- und Trainingsanweisungen, in einfacher Weise und mit hoher Genauigkeit möglich ist. Darüber hinaus soll die individuelle Leistungs- und Stoffwechseldiagnostik bei kürzerer Testdauer und höherem Komfort für den Probanden möglich sein, wobei die körperliche Belastung des Probanden möglichst gering sein soll.

Zur Lösung der vorgenannten Aufgaben wird erfindungsgemäß ein Verfahren nach Anspruch 1 sowie ein System nach Anspruch 10 vergeschlagen.

Bei dem Verfahren ist abweichend zum Stand der Technik keine kontinuierliche, sondern eine diskontinuierliche Bestimmung der physiologischen Parameter der Ventilation und/oder des Gasaustausches, insbesondere der Bestimmung des aufgenommenen Sauerstoffvolumens und des ausgeatmeten Kohlenstoffdioxidvolumens, vorgesehen. Auf der Grundlage der bestimmten physiologischen Parameter ist dann eine tatsächlich individuelle Leistungs- und Stoffwechseldiagnostik möglich, wobei, vorzugsweise, der Proband über eine Mehrzahl von Belastungsstufen steigend belastet wird ausgehend von einem Ruhezustand bis hin zu einem hohen Belastungszustand, bei dem die anaerobe Schwelle erreicht oder bereits überschritten worden ist. Durch die Bestimmung der physiologischen Parameter lediglich am Ende einer jeweiligen Belastungsstufe, wobei sich, vorzugsweise, jede Belastungsstufe über eine gleich lange Zeitdauer erstreckt, wird die Anzahl der erforderlichen Messungen verringert und gleichwohl eine exakte Bestimmung des Verlaufs des Energiestoffwechsels bei steigender Belastung erreicht. Dies trägt zu einem hohen Benutzerkomfort und einer kurzen Gesamtdauer bei der Energiestoffwechseldiagnostik bei.

Das Verfahren und das Spiroergometriesystem ermöglichen die Ermittlung eines persönlichen Energiestoffwechsels, d. h. der individuellen Bereitstellung von Fetten und Kohlenhydraten zur Energiegewinnung in der Muskulatur. Dadurch lassen sich zielgerichtet individuelle Ernährung-, Bewegungs- und Trainingsanweisungen erstellen. Es wird zu einem bestimmten Zeitpunkt und über eine kurze Zeit insbesondere die Menge des ein- und ausgeatmeten Sauerstoffs und des Kohlenstoffdioxids bestimmt. Ziel dabei kann es sein, diejenige Belastungsintensität zu ermitteln, bei der beispielsweise das Optimum des aeroben, also sauerstoff-basierten Fettstoffwechsels, erreicht ist. An diesem Punkt erfolgt die Energiegewinnung zu einem maximalen Anteil durch die Verbrennung von körpereigenen Fetten. Durch ein Training in diesem Belastungsbereich wird die Fähigkeit des Organismus verbessert, Energie aus Fetten bereitzustellen. Zur Kontrolle der Trainingsintensität orientiert sich der Anwender dann an derjenigen Herzfrequenz, die beim ermittelten Optimum der Fettverbrennung zeitgleich gemessen worden ist. Vorzugsweise kann diejenige Belastungsintensität automatisch ermittelt werden, bei der die anaerobe Schwelle erreicht ist.

Das Verfahren und das entsprechende Spiroergometriesystem haben den Vorteil, dass die Ausbelastung des Probanden bei der Bestimmung des individuellen Energiestoffwechselverlaufs nicht zwingend erforderlich ist. Der Energiestoffwechselverlauf wird bei Einsatz des Verfahrens nicht auf der Grundlage der maximalen Sauerstoffaufnahme zurückgerechnet, sondern tatsächlich (diskontinuierlich) bei vorzugsweise submaximaler Belastung des Probanden gemessen. Dadurch wird eine hohe Genauigkeit bei der Ermittlung eines persönlichen Energiestoffwechsels sichergestellt, ohne dass eine statistische Abschätzung vorgenommen werden muß.

Durch die diskontinuierliche Messung lediglich im Bereich des Endes einer Belastungsstufe ist es weiter nicht erforderlich, dass der Proband über die gesamte Dauer einer Belastungsstufe eine Atemmaske oder dergleichen trägt. Insbesondere können aufgrund der Spotmessungen am Ende der Belastungsstufen solche Anwendereinheiten zum Einsatz kommen, die aus einem Gerätegehäuse und einem (auswechselbaren) Atemrohr mit einer Absaugstelle für die Atemgasentnahme bestehen, wobei ein Verschluß der Nase zum Ausschluß der Nasenatmung des Probanden bei der Entnahme einer Gasprobe aus der Anwendereinheit vorgesehen sein kann. Das Tragen einer Gesichtsmaske ist bei Einsatz von Anwendereinheiten der vorbeschriebenen Art überhaupt nicht mehr erforderlich, was zu einem hohen Testkomfort für den Probanden führt.

Im Ergebnis ermöglichen das Verfahren und das erfindungsgemäße Spiroergometriesystem eine sehr schnelle, einfache und exakte Bestimmung des individuellen Ist-Zustandes des Energiestoffwechsels eines Probanden bei einer vergleichsweise geringen Anzahl von Messungen.

Als Parameter des Gasaustausches werden im Sinne des Verfahrens vorzugsweise die Sauerstoffaufnahme, die Kohlenstoffdioxidabgabe beim Ausatmen und/oder daraus abgeleitete Parameter, wie die anaerobe Schwelle, der respiratorische Quotient und/oder der Sauerstoffpuls bestimmt. Als Parameter der Ventilation können vorzugsweise das Atemzugvolumen, die Atemfrequenz und das Atemzeitvolumen und/oder das daraus abgeleitete Atemäquivalent für Sauerstoff bestimmt werden.

Bei einer Cardiogeräteeinheit im Sinne der Erfindung kann es sich um ein Ergometer, ein Fitneßgerät, einen Crosstrainer, ein Ruderergometer, ein Rudergerät, ein Laufband, ein Walker-Gerät, ein Spin-Bike oder ein Fahrrad oder dergleichen handeln.

Um den persönlichen Energiestoffwechselverlauf bei zunehmender Belastung zu bestimmen, kann es bereits ausreichen, die Messung der Atemgaszusammensetzung und/oder des Atemflussvolumens und die Bestimmung der physiologischen Parameter automatisch über einen Zeitraum von weniger als 40 Sekunden, vorzugsweise von weniger als 30 Sekunden, insbesondere von weniger als 10 bis 20 Sekunden, vor dem Ende einer Belastungsstufe vorzusehen. Die physiologischen Parameter können automatisch insbesondere für die letzten acht, vorzugsweise für die letzten fünf oder für weniger als fünf Atemzüge vor dem Ende einer Belastungsstufe bestimmt werden. Die Messung der Atemgaszusammensetzung und/oder des Atemflussvolumens zur Bestimmung der gewünschten physiologischen Parameter sollte jedoch erst dann erfolgen, wenn der Energiestoffwechsel einen stabilen oder gleichbleibenden Zustand erreicht hat. Im stabilen Zustand des Energiestoffwechsels könnte bei einer gleichbleibenden Belastung des Probanden grundsätzlich auch bereits ein Atemzug ausreichen, um die gewünschten physiologischen Parameter mit hoher Genauigkeit für den jeweiligen Belastungsbereich zu bestimmen. Im Ergebnis läßt es das Verfahren zu, bei einer geringen Anzahl von durchgeführten Messungen sehr exakte Angaben zum Energiestoffwechsel des Probanden zu machen.

Um eine kurze Gesamtdauer bei der Ermittlung des Energiestoffwechselverlaufs in Abhängigkeit von der Belastung des Probanden sicherzustellen, kann die Dauer einer Belastungsstufe weniger als 3 Minuten, vorzugsweise ca. 2 Minuten oder auch weniger, betragen. Die Belastungserhöhung zwischen zwei benachbarten Belastungsstufen kann zwischen 10 Watt bis 100 Watt, vorzugsweise ca. 25 Watt, betragen, so dass eine exakte Bestimmung des Energiestoffwechselverlaufs in Abhängigkeit von der vom Probanden bei Betätigung der Cardiogeräteeinheit erbrachten Leistung sichergestellt ist. In diesem Zusammenhang hat sich gezeigt, dass ein stabiler Stoffwechselzustand sich beispielsweise nach einer Zeitdauer von mehr als 60 Sekunden, vorzugsweise von mehr als 75 Sekunden, insbesondere nach ca. 90 Sekunden, einstellen kann, so dass anschließend die Messung von Atemgaszusammensetzung und/oder Atemflussvolumen und die Bestimmung der physiologischen Parameter erfolgen können.

Im Meßzeitraum vor dem Ende der jeweiligen Belastungsstufe können die physiologischen Parameter für jeden Atemzug im Meßzeitraum einzeln bestimmt und anschließend die Mittelwerte der bestimmten physiologischen Parameter über die Gesamtzahl der im Meßzeitraum vor dem Ende der Belastungsstufe getätigten Atemzüge automatisch ermittelt werden. Dies trägt zu einer hohen Genauigkeit bei der Bestimmung der Energiestoffwechsel-Parameter bei. Im übrigen kann vorgesehen sein, dass fehlerhafte Atemzüge automatisch erkannt und von der Mittelwertbildung ausgeschlossen werden. Ein "fehlerhafter" Atemzug kann beispielsweise aus der Abweichung des Kurvenverlaufs der gemessenen Sauerstoff- und/oder Kohlendioxidkonzentrationswerte und/oder des gemessenen Atemvolumenstroms von einem erwarteten Kurvenverlauf ermittelt werden.

Eine die Belastung des Probanden bestimmende Leistungseinheit der Cardiogeräteeinheit, beispielsweise eine Widerstands- oder Bremsanordnung der Cardiogeräteeinheit, kann manuell oder, vorzugsweise, automatisch gesteuert und/oder geregelt werden, weiter vorzugsweise in Abhängigkeit von vorab bestimmten physiologischen Parametern.

Zur Bestimmung derjenigen Belastungsintensität, bei der das Optimum des Fettstoffwechsels erreicht wird, ist eine maximale Ausbelastung des Probanden nicht zwingend erforderlich. Die Bestimmung der physiologischen Parameter kann in diesem Zusammenhang lediglich bei Belastungsstufen erfolgen, die zu einem respiratorischen Quotienten von maximal ca. 1,1, vorzugsweise von ca. 1,0, führen. Die Bestimmung der maximalen Sauerstoffaufnahme bei Ausbelastung des Probanden ist dann nicht vorgesehen. Dadurch werden die Gesamttestdauer bei der Analyse des Energiestoffwechsels und die körperlichen Anforderungen an den Probanden deutlich verringert.

Die Erfindung sieht vor, dass bis zum Erreichen eines respiratorischen Quotienten von kleiner als 1,09, vorzugsweise von ca. 1,0, eine stufenweise Belastungssteigerung vorgesehen und die physiologischen Parameter im Bereich des Endes der jeweiligen Belastungsstufe ermittelt werden und dass bei Erreichen eines größeren respiratorischen Quotienten die Belastung anschließend stufenlos erhöht und die physiologischen Parameter kontinuierlich bestimmt werden. Bei dieser Ausführungsform des erfindungsgemäßen Verfahrens beinhaltet die individuelle Leistungs- und Stoffwechseldiagnostik über die Bestimmung der Energiestoffwechsel-Parameter bei submaximaler Belastung auf der Grundlage eines Rampentests mit stufenförmigem Anstieg der Belastung und diskontinuierlicher Messung die anschließende Ermittlung der maximalen Sauerstoffaufnahme bei Ausbelastung des Probanden und kontinuierlicher Messung. Dadurch ist eine sehr weitgehende Leistungsdiagnostik des Energiestoffwechsels des Probanden möglich.

In der nachfolgenden Tabelle sind exemplarisch die ermittelten Werte für den Energiestoffwechsel eines Probanden bei unterschiedlicher Belastung des Probanden dargestellt. Die Messung der Atemgaszusammensetzung und/oder des Atemflussvolumens und die Bestimmung der physiologischen Parameter "Sauerstoffaufnahme" und "Kohlendioxidabgabe" bzw. "respiratorischer Quotient RER" und daraus abgeleitet die Angaben für den Kalorienverbrauch beziehen sich auf einen Meßzeitraum von maximal 30 s vor dem Ende der jeweiligen Belastungsstufe.

**Tabelle 1: Energiestoffwechselverlauf bei steigender Belastung des Probanden**

| Stufe | Leistung [watt] | Dauer | RER [] | Fett [kCal/h] | Kohlenhyd. [kCal/h] | % Fett [%] | % Kohlenhyd. [%] | HF [1/min] | Energie [kCal/h] |
|---|---|---|---|---|---|---|---|---|---|
| Ruhe | 0,0 | 00:00:57 | 0,85 | 121 | 117 | 50 | 50 | 72 | 231 |
| 1 | 75,0 | 00:02:01 | 0,81 | 242 | 139 | 63 | 37 | 80 | 383 |
| 2 | 125,0 | 00:02:00 | 0,85 | 273 | 242 | 51 | 49 | 95 | 546 |
| 3 | 175,0 | 00:01:59 | 0,86 | 300 | 352 | 46 | 54 | 105 | 655 |
| 4 | 225,0 | 00:02:01 | 0,89 | 299 | 509 | 37 | 63 | 121 | 814 |
| 5 | 275.0 | 00:01:58 | 0,91 | 291 | 687 | 30 | 70 | 134 | 979 |
| 6 | 325,0 | 00:01:59 | 0,96 | 168 | 967 | 15 | 85 | 145 | 1144 |
| 7 | 375.0 | 00:02:01 | 1,03 | 0 | 1339 | 0 | 100 | 157 | 1339 |
| 8 | 425,0 | 00:02:00 | 1.07 | 0 | 1476 | 0 | 100 | 165 | 1476 |
| 9 | 475,0 | 00:01:58 | 1.06 | 0 | 1447 | 0 | 100 | 167 | 1447 |

Der Proband zeigt gemäß Tabelle 1 eine sehr gute Grundlagenausdauer. Über die Feststoffwechselbereiche lassen sich vor allem der regenerative Trainingsbereich bis zu einem Puls von ca. 102 bei 140 Watt und der extensive Grundlagenbereich bis zu einem Puls von ca. 134 bei 275 Watt abbilden. Intensives Training erfolgt bei einer Herzfrequenz von 134 bis 152 (275 bis 350 Watt). Das sehr intensive Training im Übergangsbereich zu anaerober Energiebereitstellung sollte über eine Herzfrequenz von 152 mit mehr als 350 Watt erfolgen. Die maximale Herzfrequenz liegt bei etwa 167, was auch niedrige Trainingsherzfrequenzen erklärt.

Aus den in Tabelle 1 ermittelten physiologischen Parameterwerten lassen sich individuelle Trainingsbereiche angeben, so wie nachfolgend in Tabelle 2 dargestellt.

**Tabelle 2: individuelle Trainingsbereiche**

| | TB1 | TB2 | TB3 | TB4 |
|---|---|---|---|---|
| HF | < 108 | 108-134 | 134-153 | > 153 |
| Watt | < 150 | 150-275 | 275-358 | > 358 |

Der erste Trainingsbereich TB 1 "Regeneration" stellt einen Bereich mit individuelle hohem aktiven Feststoffwechsel (relativer Anteil an der Energiebereitstellung) dar. Der Bereich TB2 "Grundlagentraining" umfaßt das extensive Grundlagentraining mit niedriger Intensität zur Verbesserung des aeroben Stoffwechsels. Individuell liegt eine hohe Fettverbrennung vor. Im dritten Bereich TB3 "Aufbau" ist das intensive Grundlagentraining mit höherer Intensität zur Verbesserung der aeroben Leistungsfähigkeit und der Leistungsfähigkeit des Herz-Kreislauf-Systems umfaßt. Hier kommt es zum Übergang zur intensiven Kohlenhydratverbrennung. Der vierte Bereich TB4 "Wettkampf und Spitzenbereich" betrifft den sehr intensiven Trainingsbereich im Übergang zur anaeroben Energiebereitstellung, wie bei einem Intervalltraining oder einem Tempotraining.

Der ermittelte Energiestoffwechselverlauf kann graphisch in Abhängigkeit von der Belastungsintensität dargestellt werden. Aus dem gemessenen Energiestoffwechsel, insbesondere aus dem Fettstoffwechsel, können individuelle Trainingsbereiche bestimmt werden, entweder manuell oder automatisch.

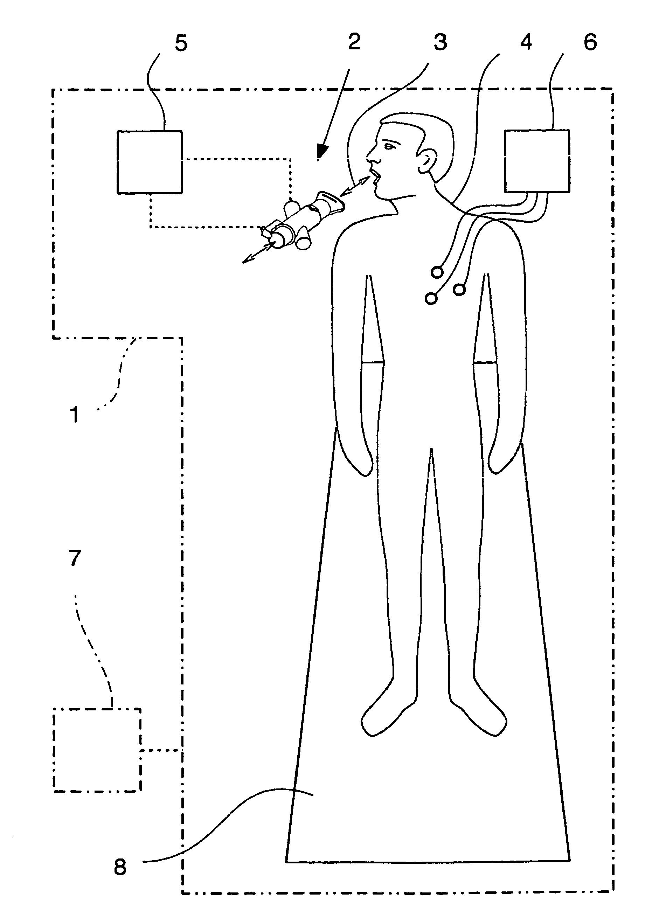

Im Einzelnen gibt es eine Vielzahl von Möglichkeiten, das erfindungsgemäße Verfahren auszugestalten und weiterzubilden, wobei einerseits auf die abhängigen Patentansprüche und andererseits auf die nachfolgende Beschreibung einer bevorzugten Ausführungsform der Erfindung unter Bezugnahme auf die Zeichnung verwiesen wird.

In der Figur ist schematisch ein Verfahren zur Steuerung und/oder Regelung eines Spiroergometriesystems 1 zur Bestimmung des Energiestoffwechsels während körperlichen Belastung gezeigt. Das Spiroergometriesystem 1 weist eine im Strom der Inspirations- und Expirationsluft eines Probanden 4 angeordnete Anwendereinheit 2 zur Entnahme einer Atemgasprobe 3 eines Probanden 4 und wenigstens eine schematisch dargestellte Analyseeinheit 5 zur Messung der Atemgaszusammensetzung und/oder des Atemflussvolumens und zur Bestimmung physiologischer Parameter der Ventilation und/oder des Gasaustausches des Probanden 4, insbesondere zur Bestimmung des bei einem Atemzug von dem Probanden 4 aufgenommenen Sauerstoffvolumens und des nach dem Atemzug ausgeatmeten Kuhlenstoffdioxidvolumens und, vorzugsweise, des respiratorischen Quotienten, auf. Darüber hinaus ist eine Messeinrichtung 6 zur Messung der Herzfrequenz des Probanden 4 vorgesehen, was ebenfalls lediglich schematisch in der Figur dargestellt ist. Schließlich weist das Spiroergometriesystem 1 eine Steuereinrichtung 7 zur Steuerung des Spiroergometriesystems 1 und eine Cardiogeräteeinheit 8 zur körperlichen Belastung des Probanden 4 auf.

Bei dem dargestellten Verfahren erfolgt bei stufenweise ansteigender Belastung des Probanden 4 bei Betätigung der Cardiogeräteeinheit 8 lediglich im Bereich des zeitlichen Endes einer Belastungsstufe vor einer Belastungssteigerung vorzugsweise automatisch eine Messung der Atemgaszusammensetzung und/oder des Atemflussvolumens und die Bestimmung der physiologischen Parameter. Insbesondere erfolgt eine Bestimmung des aufgenommenen Sauerstoffvolumens und des ausgeatmeten Kohlenstoffdioxidvolumens, bezogen auf wenigstens einen Atemzug im Bereich des Endes der Belastungsstufe. Darüber hinaus kann die Steuereinrichtung 7 derart ausgebildet sein und mit dem Spiroergometriesystem 1 zusammenwirken, dass ein Verfahren nach einem der Unteransprüche ausführbar ist.

## Patentansprüche

1. Verfahren zur Steuerung und/oder Regelung eines Spiroergometriesystems (1) zur Bestimmung des Energiestoffwechsels während körperlicher Belastung, wobei das Spiroergometriesystem (1)
- eine im Strom der Inspirations- und Expirationsluft eines Probanden angeordnete Anwendereinheit (2) zur Entnahme einer Atemgasprobe (3) des Probanden (4),
- wenigstens eine Analyseeinheit (5) zur Messung der Atemgaszusammensetzung und/oder des Atemflussvolumens und zur Bestimmung physiologischer Parameter der Ventilation und/oder des Gasaustausches des Probanden (4), nämlich zur Bestimmung des bei einem Atemzug von dem Probanden (4) aufgenommenen Sauerstoffvolumens und des nach dem Atemzug ausgeatmeten Kohlenstoffdioxidvolumens und des respiratorischen Quotienten,
- wenigstens eine Messeinrichtung (6) zur Messung der Herzfrequenz des Probanden (4),
- wenigstens eine Steuereinrichtung (7) zur Steuerung des Spiroergometriesystems (1) und
- eine Cardiogeräteeinheit (8) zur körperlichen Belastung des Probanden (4) aufweist,
- wobei bei stufenweise ansteigender Belastung des Probanden (4) bei Betätigung der Cardiogeräteeinheit (8) lediglich im Bereich des Endes einer Belastungsstufe vor einer Belastungssteigerung eine Messung der Atemgaszusammensetzung und/oder des Atemflussvolumens und die Bestimmung der physiologischen Parameter erfolgt, nämlich eine Bestimmung des aufgenommenen Sauerstoffvolumens und des ausgeatmeten Kohlenstoffdioxidvolumens, bezogen auf wenigstens einen Atemzug im Bereich des Endes der Belastungsstufe,
- wobei bis zum Erreichen eines respiratorischen Quotienten von kleiner als 1,09 eine stufenweise Belastungssteigerung vorgesehen und die physiologischen Parameter im Bereich des Endes der jeweiligen Belastungsstufe diskontinuierlich ermittelt werden und wobei bei Erreichen eines größeren respiratorischen Quotienten die Belastung anschließend stufenlos erhöht und die physiologischen Parameter kontinuierlich bestimmt werden.

2. Verfahren nach Anspruch 1, **dadurch gekennzeichnet, dass** die Messung der Atemgaszusammensetzung und/oder des Atemflussvolumens und die Bestimmung der physiologischen Parameter automatisch über einen Zeitraum von weniger als 40 Sekunden, vorzugsweise von weniger als 30 Sekunden, insbesondere von weniger als 10 bis 20 Sekunden, vor dem Ende der Belastungsstufe erfolgt.

3. Verfahren nach einem der vorhergehenden Ansprüche, **dadurch gekennzeichnet, dass** die physiologischen Parameter für weniger als 8 Atemzüge vor dem Ende der Belastungsstufe automatisch bestimmt werden.

4. Verfahren nach einem der vorhergehenden Ansprüche, **dadurch gekennzeichnet, dass** die Dauer einer Belastungsstufe weniger als 3 min. beträgt.

5. Verfahren nach einem der vorhergehenden Ansprüche, **dadurch gekennzeichnet, dass** die Belastungserhöhung zwischen zwei benachbarten Belastungsstufen zwischen 10 W und 100 W beträgt.

6. Verfahren nach einem der vorhergehenden Ansprüche, **dadurch gekennzeichnet, dass** die physiologischen Parameter für jeden Aternzug vor dem Ende der Belastungsstufe einzeln bestimmt und anschließend die Mittelwerte der physiologischen Parameter über die Gesamtzahl der im Messzeitraum vor dem Ende der Belastungsstufe getätigten Aternzüge automatisch gebildet werden.

7. Verfahren nach einem der vorhergehenden Ansprüche, dadurch gekennzeichniet, dass fehlerhafte Atemzüge automatisch erkannt und von der Mittelwertbildung ausgeschlossen werden.

8. Verfahren nach einem der vorhergehenden Ansprüche, **dadurch gekennzeichnet, dass** eine die Belastung des Probanden (4) bestimmende Leistungseinheit der Cardiogeräteeinheit (8) automatisch gesteuert und/oder geregelt wird.

9. Verfahren nach einem der vorhergehenden Ansprüche 1 bis 8, **dadurch gekennzeichnet, dass** bis zum Erreichen eines respiratorischen Quotienten von kleiner oder gleich 1,03 eine stufenweise Belastungssteigerung vorgesehen und die physiologischen Parameter im Bereich des Endes der jeweiligen Belastungsstufe diskontinuierlich ermittelt werden und dass bei Erreichen eines größeren respiratorischen Quotienten die Belastung anschließend stufenlos erhöht und die physiologischen Parameter kontinuierlich bestimmt werden.

10. Spiroergometriesystems (1) zur Bestimmung des Energiestoffwechsels während körperlicher Belastung, wobei das Spiroergometriesystem
- eine im Strom der Inspirations- und Expirationsluft eines Probanden (4) angeordnete Anwendereinheit (2) zur Entnahme einer Atemgasprobe des Probanden (4),
- wenigstens eine Analyseeinheit (5) zur Messung der Atemgaszusammensetzung und/oder des Atemflussvolumens und zur Bestimmung physiologischer Parameter der Ventilation und/oder des Gasaustausches des Probanden (4), nämlich zur Bestimmung des bei einem Atemzug von dem Probanden (4) aufgenommenen Sauerstoffvolumens und des nach dem Atemzug ausgeatmeten Kohlendioxidvolumens und des respiratorischen Quotienten,
- wenigstens eine Messeinrichtung (6) zur Messung der Herzfrequenz des Probanden (4),
- wenigstens eine Steuereinrichtung (7) zur Steuerung des Spiroergometriesystems und
- eine Cardiogeräteeinheit (8) zur körperlichen Belastung des Probanden (4)
aufweist und wobei die Steuereinrichtung (7) derart ausgebildet ist, dass bei stufenweise ansteigender Belastung des Probanden (4) bei Betätigung der Cardiogeräteeinheit (8) lediglich im Bereich des Endes einer Belastungsstufe vor einer Belastungssteigerung eine Messung der Atemgaszusammensetzung und/oder des Atemflussvolumens und die Bestimmung der physiologischen Parameter erfolgt, nämlich eine Bestimmung des aufgenommenen Sauerstoffvolumens und des ausgeatmeten Kohlenstoffdioxidvolumens, bezogen auf wenigstens einen Atemzug im Bereich des Endes der Belastungsstufe, dass bis zum Erreichen eines respiratorischen Quotienten von kleiner als 1,09 eine stufenweise Belastungssteigerung vorgesehen und die physiologischen Parameter im Bereich des Endes der jeweiligen Belastungsstufe diskontinuierlich ermittelt werden und dass bei Erreichen eines größeren respiratorischen Quotienten die Belastung anschließend stufenlos erhöht und die physiologischen Parameter kontinuierlich bestimmt werden.

## Claims

1. A method for controlling and/or regulating a spiroergometry system (1) for determining the energy metabolism during physical exertion, in which the spiroergometry system (1) comprises
- a user unit (2) arranged in the stream of the inspiration air and expiration air of a volunteer for taking a sample (3) of the respiratory gas of the volunteer (4),
- at least one analysis unit (5) for measuring the composition of the respiratory gas and/or the volume of air flow and for determining physiological parameters of the ventilation and/or of the gas exchange of the volunteer (4), namely for determining the oxygen volume inhaled by the volunteer (4) in one breath and the carbon dioxide volume exhaled after the breath along with the respiratory quotient,
- at least one measuring unit (6) for measuring the heart rate of the volunteer (4),
- at least one control unit (7) for controlling the spiroergometry system (1) and
- a cardio device unit (8) for physical exertion of the volunteer (4),
- wherein a measurement of the respiratory gas composition and/or the air flow volume and determination of the physiological parameters are performed while the volunteer (4) is exercising on a gradually increasing scale while operating the cardio device unit (8), said measurement begin performed only in the range of the end of an exertion stage before an increase in exertion, namely the oxygen volume inhaled and the carbon dioxide volume exhaled are determined, based on at least one breath in the range of the end of the exertion stage,
- wherein a stepwise increase in exertion is provided until achieving a respiratory quotient of less than 1.09, and the physiological parameters are determined discontinuously in the range of the end of the respective exertion stage, and wherein the exertion is then increased continuously on reaching a larger respiratory quotient, and the physiological parameters are then determined continuously.

2. The method according to Claim 1, **characterized in that** the measurement of the respiratory gas composition and/or the respiratory flow volume and the determination of the physiological parameters are performed automatically over a period of less than 40 seconds, preferably less than 30 seconds, in particular less than 10 to 20 seconds, before the end of the exertion stage.

3. The method according to any one of the preceding claims, **characterized in that** the physiological parameters are determined automatically for fewer than eight breaths before the end of the exertion stage.

4. The method according to any one of the preceding claims, **characterized in that** the duration of one exertion stage amounts to less than three minutes.

5. The method according to any one of the preceding claims, **characterized in that** the increase in exertion between two neighboring exertion stages is between 10 W and 100 W.

6. The method according to any one of the preceding claims, **characterized in that** the physiological parameters for each breath are determined individually before the end of the exertion stage and then the averages of the physiological parameters are formed automatically over the total number of breaths taken in the measurement period of time before the end of the exertion stage.

7. The method according to any one of the preceding claims, **characterized in that** breaths not performed correctly are detected automatically and excluded from the averaging.

8. The method according to any one of the preceding claims, **characterized in that** a power unit of the cardio device unit (8), which determines the exertion of the volunteer (4), is controlled and/or regulated automatically.

9. The method according to any one of the preceding Claims 1 through 8, **characterized in that** a stepwise increase in exertion is provided until reaching a respiratory quotient of less than or equal to 1.03 and the physiological parameters are determined discontinuously in the range of the end of the respective exertion step, and on reaching a higher respiratory quotient, the exertion is then increased continuously and the physiological parameters are determined continuously.

10. A spiroergometry system (1) for determining the energy metabolism during physical exertion, wherein the spiroergometry system has:
- a user unit (2) which is arranged in the stream of the inspiration air and expiration air of a volunteer (4) to take a sample of the respiratory gas of the volunteer (4),
- at least one analysis unit (5) for measuring the respiratory gas composition and/or the respiratory flow volume and for determining physiological parameters of ventilation and/or the gas exchange of the volunteer (4), namely for determining the oxygen volume inhaled with a breath by the volunteer (4) and the carbon dioxide volume exhaled after the breath and for determining the respiratory quotient,
- at least one measuring unit (6) for measuring the heart rate of the volunteer (4),
- at least one control unit (7) for controlling the spiroergometry system and
- a cardio device unit (8) for physical exertion of the volunteer (4),
and wherein the control unit (7) is designed so that with a stepwise increase in the exertion of the volunteer (4) on activation of the cardio device unit (8), a measurement of the respiratory gas composition and/or of the respiratory flow volume and the determination of the physiological parameters are performed only in the range of the end of an exertion stage before an increase in exertion, namely there is a determination of the oxygen volume inhaled and the carbon dioxide volume exhaled, based on at least one breath in the range of the end of the exertion stage, such that a stepwise increase in exertion is provided until reaching a respiratory quotient of less than 1.09, and the physiological parameters are determined discontinuously in the range of the end of the respective exertion stage, and on reaching a larger respiratory quotient, the exertion is then increased continuously and the physiological parameters are determined continuously.

## Revendications

1. Procédé de commande et/ou de réglage d'un système de spiroergométrie (1) pour la définition du métabolisme énergétique pendant l'effort physique, dans lequel le système de spiroergométrie (1) présente
- une unité d'utilisateur (2) disposée dans le flux de l'air inspiré et expiré d'une personne testée et servant à prélever un échantillon d'air respiratoire (3) de la personne testée (4),
- au moins une unité d'analyse (5) pour mesurer la composition du gaz respiratoire et/ou le volume de flux respiratoire et pour définir des paramètres physiologiques de ventilation et/ou d'échange gazeux de la personne testée (4), à savoir pour définir le volume d'oxygène absorbé par la personne testée (4) lors d'une respiration et le volume de dioxyde de carbone expiré après la respiration et le quotient respiratoire,
- au moins un dispositif de mesure (6) pour mesurer la fréquence cardiaque de la personne testée (4),
- au moins un dispositif de commande (7) pour commander le système de spiroergométrie (1) et
- une unité d'appareillage cardiaque (8) pour faire faire un effort physique à la personne testée (4),
- une mesure de la composition du gaz respiratoire et/ou du volume de flux respiratoire et la définition des paramètres physiologiques étant réalisées simplement vers la fin d'un cycle d'effort avant une augmentation de l'effort, à savoir une définition du volume d'oxygène absorbé en se référant à au moins une respiration vers la fin d'un cycle d'effort,
- étant prévue jusqu'à l'atteinte d'un quotient respiratoire inférieur à 1,09 une augmentation graduelle de l'effort et les paramètres physiologiques étant déterminés de manière discontinue vers la fin du cycle d'effort respectif et, en cas d'atteinte d'un quotient respiratoire supérieur, l'effort étant ensuite augmenté graduellement et les paramètres physiologiques étant déterminés de manière continue.

2. Procédé selon la revendication 1, **caractérisé en ce que** la mesure de la composition du gaz respiratoire et/ou du volume de flux respiratoire et la définition des paramètres physiologiques sont réalisées automatiquement sur une période de moins de 40 secondes, de préférence de moins de 30 secondes, en particulier de moins de 10 à 20 secondes, avant la fin du cycle d'effort.

3. Procédé selon une des revendications précédentes, **caractérisé en ce que** les paramètres physiologiques sont définis automatiquement pour moins de 8 respirations avant la fin du cycle d'effort.

4. Procédé selon une des revendications précédentes, **caractérisé en ce que** la durée d'un cycle d'effort est de moins de 3 minutes.

5. Procédé selon une des revendications précédentes, **caractérisé en ce que** l'augmentation de l'effort entre deux cycles d'effort voisins est comprise entre 10 W et 100 W.

6. Procédé selon une des revendications précédentes, **caractérisé en ce que** les paramètres physiologiques pour chaque respiration sont définis individuellement avant la fin du cycle d'effort puis que les valeurs moyennes des paramètres physiologiques sont établies à l'aide du nombre total de respirations faites dans la période de mesure précédant la fin du cycle d'effort.

7. Procédé selon une des revendications précédentes, **caractérisé en ce que** les respirations défaillantes sont automatiquement détectées et exclues du calcul de moyenne.

8. Procédé selon une des revendications précédentes 1 à 8, **caractérisé en ce qu'**une unité de puissance définissant l'effort de la personne testée (4) de l'unité d'appareillage cardiaque (8) est automatiquement commandée et/ou réglée.

9. Procédé selon une des revendications précédentes 1 à 8, **caractérisé en ce que**, jusqu'à l'atteinte d'un quotient respiratoire inférieur ou égal à 1,03, une augmentation graduelle de l'effort est prévue et les paramètres physiologiques sont déterminés de manière discontinue vers la fin du cycle d'effort respectif et que, en cas d'atteinte d'un quotient respiratoire supérieur, l'effort est ensuite augmenté progressivement et les paramètres physiologiques sont déterminés de manière continue.

10. Système de spiroergométrie (1) pour la définition du métabolisme énergétique pendant l'effort physique, dans lequel le système de spiroergométrie présente
- une unité d'utilisateur (2) disposée dans le flux de l'air inspiré et expiré d'une personne testée (4) et servant à prélever un échantillon d'air respiratoire de la personne testée (4),
- au moins une unité d'analyse (5) pour mesurer la composition du gaz respiratoire et/ou le volume de flux respiratoire et pour définir des paramètres physiologiques de ventilation et/ou d'échange gazeux de la personne testée (4), à savoir pour définir le volume d'oxygène absorbé par la personne testée (4) lors d'une respiration et le volume de dioxyde de carbone expiré après la respiration et le quotient respiratoire,
- au moins un dispositif de mesure (6) pour mesurer la fréquence cardiaque de la personne testée (4),
- au moins un dispositif de commande (7) pour commander le système de spiroergométrie et
- une unité d'appareillage cardiaque (8) pour faire faire un effort physique à la personne testée (4),
l'unité de commande (7) étant réalisée de manière à ce que, lors de l'actionnement de l'unité d'appareillage cardiaque (8) simplement vers la fin d'un cycle d'effort avant une augmentation de l'effort, une mesure de la composition du gaz respiratoire et/ou du volume de flux respiratoire et la définition des paramètres physiologiques aient lieu, à savoir une définition du volume d'oxygène absorbé et du volume de dioxyde de carbone expiré, en se référant à au moins une respiration vers la fin d'un cycle d'effort, sachant que, pour atteindre un quotient respiratoire inférieur à 1,09, une augmentation graduelle de l'effort est prévue et que les paramètres physiologiques sont déterminés de manière discontinue vers la fin du cycle d'effort respectif et que, en cas d'atteinte d'un quotient respiratoire supérieur, l'effort est ensuite augmenté graduellement et les paramètres physiologiques sont déterminés de manière continue.
